# EUROPEAN PATENT APPLICATION

(11) **EP 2 000 139 A1**
(43) Date of publication of application: **10.12.2008**
(21) Application number: 07109816.4
(22) Date of filing: 07.06.2007
(51) Int. Cl.: A61K 31/506, A61K 9/16, A61P 35/02

(54) **Stabilized amorphous forms of imatinib mesylate**

(71) Applicant: NOVARTIS AG, 4002 Basel (CH)
(72) Inventor: The designation of the inventor has not yet been filed
(74) Representative: Roth, Peter Richard

(57) **Abstract**

The invention relates to the stabilized amorphous form of the methanesulfonic acid addition salt of 4-(4-methylpiperazin-1-ylmethyl)-N-[4-methyl-3-(4-(pyridin-3-yl)-pyrimidin-2-ylamino)-phenyl]-benzamide, pharmaceutical compositions such as capsules or tablets containing this form, the use of such form in diagnostic methods or, preferably, for the therapeutic treatment of warm-blooded animals, especially humans, and the use of formulation principles stabilizing the amorphous form of Imatinib mesylate as an intermediate for the preparation of pharmaceutical compositions.

## Description

The invention relates to the stabilized amorphous form of the methanesulfonic acid addition salt of 4-(4-methylpiperazin-1-ylmethyl)-N-[4-methyl-3-(4-(pyridin-3-yl)-pyrimidin-2-ylamino)-phenyl]-benzamide, pharmaceutical compositions containing this form, the use of such form in diagnostic methods or, preferably, for the therapeutic treatment of warm-blooded animals, especially humans, and the use of formulation principles stabilizing the amorphous form of Imatinib mesylate as an intermediate for the preparation of pharmaceutical compositions.

### Background to the invention

It is well known that molecules can arrange to form different crystal polymorphs. Polymorphs have the same composition, but exhibit different solid-state properties as e.g. stability or solubility.

The preparation of 4-(4-methylpiperazin-1-ylmethyl)-N-[4-methyl-3-(4-(pyridin-3-yl)-pyrimidin-2-ylamino-)phenyl]-benzamide, also known as Imatinib, and its use, especially as an anti-tumour agent, are described in Example 21 of US 5,521,184. The compound is exemplified in these publications only in free form (not as a salt).

4-(4-Methylpiperazin-1-ylmethyl)-N-[4-methyl-3-(4-(pyridin-3-yl)-pyrimidin-2-ylamino)-phenyl]-benzamide mesylate, also known as Imatinib mesylate or STI571, as well as the alpha and the beta crystal form thereof are described in US 6,894,051. Imatinib mesylate is the active ingredient of the drug Gleevec® (Glivec®) which is an approved medicament for the treatment of Chronic Myeloid Leukemia (CML) and gastrointestinal stromal tumors (GIST) as well as a number of rare proliferative disorders.

In US 6,894,051 it is reported that the beta crystal modification of Imatinib mesylate is thermodynamically more stable than the alpha form at temperatures below 140 °C. Furthermore, both forms are thermodynamically more stable than the amorphous form of Imatinib mesylate. The different stabilities have the effect that the meta-stable amorphous form of Imatinib mesylate bears the risk of conversion into a more stable modification such as the alpha or the beta crystal form.

It was now surprisingly found that the amorphous form of Imatinib mesylate can be stabilized by various formulation principles. Hence, in a first aspect the present invention relates to formulation principles that stabilize the amorphous form of Imatinib mesylate. More specifically, the present invention describes various formulation principles capable of stabilizing the amorphous form of Imatinib mesylate including solid dispersions, cyclodextrin complexes, and co-milling with excipients.

The term "stabilizing Imatinib mesylate in the amorphous form" as used herein means especially that Imatinib mesylate is maintained in the amorphous form after 1 month of storage at 40°C at 75% relative humidity. The term "Imatinib mesylate in the amorphous form" designates a modification of Imatinib mesylate, which does not show any reflexes in X-ray diagrams that would correlate to reflexes observed for a crystalline modification of Imatinib mesylate , especially the alpha or beta crystal form of Imatinib mesylate.

Compared to crystalline forms of Imatinib mesylate, a stabilized amorphous form of Imatinib mesylate offers a number of advantages including economic advantages and a higher dissolution rate.

Crystalline material is generally obtained through a crystallization process, which constitutes an additional manufacturing step. In particular, complete crystallization from a mother liquor is often a time consuming process step, thus binding production capacity. Therefore, a stabilized amorphous form of Imatinib mesylate is an attractive alternative to crystalline forms under economic aspects.

In general, amorphous forms of a substance show a higher dissolution rate than crystalline forms of the same substance. Furthermore, a high dissolution rate can result in oversaturated solutions. The higher dissolution rate as well as the potentially obtained oversaturated solution can also result in better bioavailability of the amorphous form of a substance compared to a crystalline form thereof. The same amount of drug could thus be absorbed by a patient obtaining a lower dose of a given drug. This lowers the risk of local side effects in the patients caused by not absorbed materials and also has a cost saving effect.

### Detailed description of the invention

The invention relates especially to formulation principles that stabilize the amorphous form of Imatinib mesylate. The formulation principle according to the present invention is especially selected from solid dispersions, cyclodextrin complexes, and co-milling with selected excipients. The formulation principles described herein can be used as starting materials for the manufacture of pharmaceutical compositions, such as tablets, suspensions, powders, sachets, capsules or suppositories, comprising amorphous Imatinib mesylate. Depending on the specific composition used, these compositions can, e.g., be applied oral, rectal, vaginal or by inhalation.

### 1. Solid dispersions

The solid dispersions of the present invention comprise amorphous Imatinib mesylate and at least one further excipient selected from cellulose derivatives, polyvinylpyrrolidone, polyethyleneglycols of various molecular weights, polyethylene-/polypropylene-/polyethylene-oxide block copolymers and polymethacrylates. Representative examples of cellulose derivatives include hydroxypropylmethylcellulose (HPMC), hydroxypropylcellulose (HPC), methylcellulose (MC), cellulose acetate phthalate (CAP), hydroxypropylmethylcellulose phthalate (HPMC-P), hydroxylpropyl methylcellulose acetate succinate (HPMC-AS), carboxymethylethylcellulose (CMEC). Other suitable excipients in solid dispersion formulations include, but are not limited to, polyvinylalcohol (PVA) and co-polymers thereof with PVP or with other polymers, polyacrylates, urea, chitosan and chitosan glutamate, sorbitol or other polyols such as mannitol.

Optionally, the solid dispersions comprise additionally at least one surfactant such as sodium dodecyl sulfate (SDS), polyoxyethylene sorbitan fatty acid esters such as Tween® 80, bile salts such as sodium deoxycholate, polyoxyethylene mono esters of a saturated fatty acid such as Solutol® HS 15, water soluble tocopheryl polyethylene glycol succinic acid esters such as Vitamin E TPGS.

The solid dispersions may contain amorphous Imatinib Mesylate in an amount by weight of the composition of about 0.01 % to about 80%; for example, in an amount by weight of about 0.01% to about 80%, 0.1% to about 70%, such as 1% to 60%, for example 2%, 5%, 10%, 20%, 30%, 40%, 50%, or 60%. The polymeric excipient may be present in an amount from about 0.1 % to 99.99% by weight of the composition. When a surfactant is present, it may generally be present in an amount of from about 0.01% to about 30%, for example from about 1 % to about 20% by weight, e.g. 1 % to 15% by weight such as 5% to 15% by weight of the composition.

In one embodiment of the invention, the cellulose derivative is selected from hydroxypropylcellulose (HPC), hydroxypropylmethylcellulose (HPMC) and hydroxypropylmethylcellulose acetate succinate (HPMC-AS). The solid dispersion in such case comprises preferably between 50 and 90 % by weight of the cellulose derivative and 10 to 50 % by weight of amorphous Imatinib mesylate.

If polyvinylpyrrolidone is employed as further excipient in the solid dispersion, the solid dispersion preferably comprises between 50 and 90 % by weight of polyvinylpyrrolidone and 10 to 50 % by weight of amorphous Imatinib mesylate.

Suitable polyethyleneglycols are especially Polyethyleneglycol 8000 and Polyethyleneglycol 6000. The solid dispersion preferably comprises between 50 and 90 % by weight of a polyethyleneglycol and 10 to 50 % by weight of amorphous Imatinib mesylate.

A suitable polyethylene-/polypropylene-/polyethylene-oxide block copolymer is in particular Pluronic F68. The solid dispersion preferably comprises between 50 and 90 % by weight of a polyethylene-/polypropylene-/polyethylene-oxide block copolymer and 10 to 50 % by weight of amorphous Imatinib mesylate.

Eudragit^{®}L-100-55 and Eudragit®E-100 are suitable polymethacrylates for the present invention. The solid dispersion preferably comprises between 70 and 90 % by weight of a polymethacrylates and 10 to 30 % by weight of amorphous Imatinib mesylate.

Optionally, surfactants can be added to solid dispersion. In such case, typically 1 to 10% by weight, preferably 2 to 4 % by weight, of the excipient mentioned above is replaced by a surfactant.

### 2. Cyclodextrin complexes

The cyclodextrin Imatinib mesylate complexes being useful as formulation principle according to the present invention comprise amorphous Imatinib mesylate, at least one cyclodextrin such as e.g. a β-cyclodextrin or an α-cyclodextrin, and optionally at least one additional excipient. Examples of suitable β-cyclodextrins include methyl-β-cyclodextrin, dimethyl-β-cyclodextrin, hyrdroxypropyl-β-cyclodextrin, glycosyl-β-cyclodexterin, maltosyl-β-cyclodextrin, sulfo-β-cyclodextrin, sulfo-alkylethers of β-cyclodextrin, e.g. sulfo-C[1-4]-alkyl ethers. Examples of α-cyclodextrins include glucosyl-α-cyclodextrin and maltosyl-α-cyclodextrin.

The cyclodextrin Imatinib mesylate complexes preferably comprise between 10 to 30 % by weight of amorphous Imatinib mesylate.

If no additional excipient is added, the cyclodextrin Imatinib mesylate complexes preferably comprise between 70 to 90 % by weight of the cyclodextrin.

In one preferred embodiment of the present invention, the cyclodextrin is selected from β-cyclodextrin and Hydroxypropyl-β-cydodextrin.

The at least one additional excipient is preferably selected from polyvinylpyrrolidone, e.g. PVPK30, cellulose derivatives, e.g. hydroxypropylcellulose (HPC), hydroxypropylmethylcellulose (HPMC) or hydroxypropylmethylcellulose acetate succinate (HPMC-AS), and surfactants, e.g. Solutol HS 15 or vitamin E TPGS.

### 3. Co-milling with excipients

In one embodiment of the present invention, the formulation principle for stabilizing amorphous Imatinib mesylate is co-milling with selected excipients. In such embodiment, amorphous Imatinib mesylate can be dry co-milled or wet co-milled with the added excipients.

For dry co-milling the added excipient can be selected from polyvinylpyrrolidone, e.g. PVPK30, cellulose derivatives, such as, but not limited to, hydroxypropylcellulose (HPC), hydroxypropylmethylcellulose (HPMC), hydroxypropylmethylcellulose acetate succinate (HPMC-AS), hydroxypropylcellulose phthalate (HPMC-P), methylcellulose (MC), polyethyleneglycols, and earth alkali metal silicas and silicates, e.g. fumed silicas, precipitated silicas, calcium silicates, such as Zeopharm^{®}600, or magnesium aluminometasilicates such as Neusilin US2.

Formulation principles obtained by dry co-milling preferably comprise between 10 to 50 %, more preferably 30 to 50 %, by weight of amorphous Imatinib mesylate.

A wet co-milled Imatinib Mesylate - excipient composition is obtained by co-milling amorphous Imatinib mesylate with the other excipients in a suitable solvent, preferably medium chain fatty acid triglycerides such as those known and commercially available under the trade names Acomed®, Myritol®, Captex®, Neobee®M 5 F, Miglyol®812, Mazol®, Sefsol®860. Miglyol®812, a fractionated coconut oil, is especially the most preferred. The other excipients can be especially a polyethylene-/polypropylene-/polyethylene-oxide block copolymers, in particular Pluronic F68 and, optionally, a small amount of a surfactant, e.g. sodium dodecyl sulfate (SDS).

A wet co-milled Imatinib Mesylate - excipient composition is obtained by co-milling amorphous Imatinib mesylate with the other excipients in a suitable solvent. Representative suitable solvents include, but are not limited to, pharmaceutically acceptable oils, preferably with an unsaturated component such as a vegetable oil; monoglycerides of medium chain fatty acids, such as Imwitor® 308 or Capmul MCM C8; medium chain fatty acid triglycerides such as those known and commercially available under the trade names Acomed®, Myritol®, Captex®, Neobee®M 5 F, Miglyol®812, Mazol®, Sefsol®860; mixed mono-di-triglycerides such as Maisine®; transesterified ethoxylated vegetable oils such as Labrafil® M 2125 CS; glycerol triacetate; polyglycerol fatty acid esters such as Plurol Oleique CC497. The other excipients can be selected from cellulose derivatives such as hydroxypropylmethylcellulose, polyvinylpyrrolidone, polyethyleneglycols, polyethylene-/polypropylene-/polyethylene-oxide block copolymers such as Pluronic F68, polymethacrylates, sodium dodecyl sulfate, polyoxyethylene sorbitan fatty acid esters such as Tween® 80, bile salts such as sodium deoxycholate, polyoxyethylene mono esters of a saturated fatty acid such as Solutol® HS 15, water soluble tocopheryl polyethylene glycol succinic acid esters such as Vitamin E TPGS. Especially, Pluronic F68 and optionally, a small amount of a surfactant, e.g. sodium dodecyl sulfate are examples of excipients that stabilize the amorphous form of Imanib mesylate upon wet co-milling.

### 4. Methods of using the stabilized amorphous form of lmatinib mesylate

Stabilized amorphous forms of Imatinib mesylate possess valuable pharmacological properties and may, for example, be used as an anti-tumour agent or as an agent to treat restenosis.

The present invention relates especially to a stabilized amorphous form of Imatinib mesylate in the treatment of one of the said diseases mentioned herein or in the preparation of a pharmacological agent for the treatment thereof.

The antiproliferative, especially anti-tumour, activity of the methanesulfonic acid addition salt of a compound of formula I *in vivo* is, for example, described for the treatment of abl-dependent tumours in Nature Med. 2, 561-6 (1996).

The invention relates also to a method for the treatment of warm-blooded animals suffering from said diseases, especially leukemia, wherein a quantity of a stabilized amorphous form of Imatinib mesylate which is effective against the disease concerned, especially a quantity with antiproliferative efficacy, is administered to warm-blooded animals in need of such treatment. The invention relates moreover to the use of a stabilized amorphous form of Imatinib mesylate for the preparation of pharmaceutical compositions for use in treating the human or animal body, especially for the treatment of tumours, such as gliomas or prostate tumours.

In preferred embodiments, the present invention relates to the use in of a stabilized amorphous form of Imatinib mesylate in the treatment of one of the disorders listed below:
1. GIST,
2. advanced chronic myeloid leukemia,
3. newly diagnosed chronic myeloid leukemia,
4. pediatric Philadelphia chromosome-positive chronic myeloid leukemia,
5. Philadelphia chromosome-positive acute lymphocytic leukemia (ALL),
6. glioblastoma multiforme, preferably in combination with hydroxyurea,
7. dermatofibrosarcoma protuberans (DFSP),
8. hypereosinophilic sindrome (HES),
9. chronic myelomonocytic leucemia (CMML), and
10. idiopathic pulmonary fibrosis.

Depending on species, age, individual condition, mode of administration, and the clinical picture in question, effective doses, for example daily doses of a stabilized amorphous form of Imatinib mesylate, which correspond to about 100-2000 mg, preferably 200-1000 mg, especially 250-800 mg of Imatinib mesylate, are administered to warm-blooded animals of about 70 kg bodyweight. Preferably, daily dosages of a stabilized amorphous form of Imatinib mesylate, which correspond to about 400 mg or 600 mg of Imatinib mesylate, are administered orally once daily, preferably together with a meal and a large glass of water (about 200 mL). Daily doses of a stabilized amorphous form of Imatinib mesylate, which correspond to about 800 mg of Imatinib mesylate are preferably administered in the form of 400 mg dosages twice daily together with food.

In one embodiment, the formulation principle is provided in the form of a capsule, which is a hard gelatine capsule containing a dry powder blend. The capsule shell preferably contains gelatine and titanium dioxide as well as red iron oxide. The ratio of weight of capsule fill to capsule shell is preferably between about 100:25 and 100:50, more preferably between 100:30 and 100:40.

In another embodiment, the formulation principle is provided in the form of a suspension comprising a stabilized amourphous Imatinib mesylate formulation obtained by co-milling amorphous Imatinib mesylate with at least one pharmaceutically acceptable excipient in a suitable solvent.

Accordingly, the present invention provides
(a) a stabilized amorphous form of Imatinib mesylate,
(b) pharmaceutical composition comprising a formulation principle that stabilizes the amorphous form of Imatinib mesylate and amorphous Imatinib mesylate, optionally together with at least one pharmaceutically acceptable excipient, especially wherein the formulation principle is selected from solid dispersions, cyclodextrin complexes, and co-milling with selected excipients;
(c) a capsule comprising a formulation principle that stabilizes the amorphous form of Imatinib mesylate and amorphous Imatinib mesylate, optionally together with at least one pharmaceutically acceptable excipient, which contains between an amount of stabilized amorphous Imatinib mesylate which corresponds to 50 mg and 200 mg of Imatinib mesylate, and, optionally wherein the shell contains gelatine and/or, wherein the shell contains titanium dioxide and/or wherein the shell contains red iron oxide. In such a capsule, the ratio of weight of capsule fill to capsule shell is between about 100:25 and 100:50, especially between 100:30 and 100:40;
(d) a tablet comprising a formulation principle that stabilizes the amorphous form of Imatinib mesylate and amorphous Imatinib mesylate, optionally together with at least one pharmaceutically acceptable excipient, in particular comprising an amount of stabilized amorphous Imatinib mesylate which corresponds to 100 mg, 400 mg or 800 mg of Imatinib mesylate;
(e) a suspension comprising a wet co-milled formulation that stabilizes the amorphous form of Imatinib mesylate and amorphous Imatinib mesylate, optionally together with at least one pharmaceutically acceptable excipient, in a suitable solvent, in particular comprising an amount of stabilized amorphous Imatinib mesylate which corresponds to 100 mg, 400 mg or 800 mg of Imatinib mesylate;
(f) the use of a stabilized amorphous form of Imatinib mesylate for the preparation of a medicament for the treatment of a disease selected from metastatic, inoperable GIST, advanced chronic myeloid leukemia, newly diagnosed chronic myeloid leukemia, pediatric Philadelphia chromosome-positive chronic myeloid leukemia, Philadelphia chromosome-positive acute lymphocytic leukemia (ALL), glioblastoma multiforme, dermatofibrosarcoma protuberans (DFSP), hypereosinophilic sindrome (HES), and chronic myelomonocytic leucemia (CMML);
(g) a method of treating a disease selected from metastatic, inoperable GIST, advanced chronic myeloid leukemia, newly diagnosed chronic myeloid leukemia, pediatric Philadelphia chromosome-positive chronic myeloid leukemia, Philadelphia chromosome-positive acute lymphocytic leukemia (ALL), glioblastoma multiforme, dermatofibrosarcoma protuberans (DFSP), hypereosinophilic sindrome (HES), and chronic myelomonocytic leucemia (CMML in a warm-blooded animal in need thereof comprising administering to the animal a stabilized amorphous form of Imatinib mesylate in a quantity which is therapeutically effective against the respective disease; and
(h) the use of a formulation principle which stabilizes the amorphous form of Imatinib mesylate as an intermediate for the preparation of a pharmaceutical composition comprising the amorphous form of Imatinib mesylate.

The following Examples illustrate the invention without limiting the scope thereof. The examples listed below describe formulations where no crystalline drug is detected after 1 mo. storage at 40°C/75% RH.

### EXAMPLE 1

This Example lists representative solid dispersion compositions of amorphous Imatinib Mesylate (Table 1) and describes making a solid dispersion according to the invention. Imatinib Mesylate (crystal form beta) is formulated as a solid dispersion using high throughput screening technology (HTS) as follows. A quantity of Imatinib Mesylate is first dissolved in a suitable solvent (95% ethanol or acetone:ethanol:water (50:40:10) to provide a stock solution (25 mg/mL). An adequate volume of this solution (40 to 200 µL) is then dispensed into each well of a 96-well plate HTS Crissy Platform to deliver the desired amount of Imatinib Mesylate (1 to 5 mg) to each well. The solvent is then evaporated to dryness. A quantity of each excipient is dissolved or suspended in a suitable solvent (95% ethanol or acetone:ethanol:water (50:40:10)) to provide a stock solution (25 mg/mL). An adequate volume of the excipient stock solution (40 to 360 µL) is then added to each well containing a quantity of Imatinib Mesylate. Contents of each well are mixed and the solvent is evaporated to dryness. The 96-well plate is scanned using X-ray powder diffraction (XRPD) to monitor the presence of crystalline Imatinib Mesylate both prior and after storage for 1 mo. at 40°C/75% RH.

**Table 1 - Representative solid dispersion compositions of amorphous Imatinib Mesylate**

| Imatinib Mesylate (wt%) | Excipient type | Excipient (wt%) | Solvent composition |
|---|---|---|---|
| **Cellulose derivatives** | | | |
| 10 | hydroxypropylcellulose (HPC) | 90 | 95% Ethanol |
| 50 | HPC | 50 | 95% Ethanol |
| 10 | hydroxypropylmethylcellulose (HPMC) | 90 | Acetone:ethanol: water (50:40:10) |
| 50 | HPMC | 50 | Acetone:ethanol: water (50:40:10) |
| 10 | hydroxypropylmethylcellulose acetate succinate (HPMC-AS) | 90 | 95% Ethanol |
| 50 | HPMC-AS | 50 | 95% Ethanol |
| **Polyvinylpyrrolidone** | | | |
| 10 | Polyvinylpyrrolidone (PVPK30) | 90 | 95% Ethanol |
| 50 | PVPK30 | 50 | 95% Ethanol |
| **Polyethyleneglycols** | | | |
| 10 | Polyethyleneglycol 6000 (PEG6000) | 90 | 95% Ethanol |
| 50 | PEG6000 | 50 | 95% Ethanol Acetone:ethanol: |
| 10 | Polyethyleneglycol 8000 (PEG8000) | 90 | water (50:40:10) |
| 50 | PEG8000 | 50 | 95% Ethanol |
| **Polyethylene-/polypropylene-/polyethylene-oxide block copolymers** | | | |
| 10 | Pluronic F68 | 90 | 95% Ethanol |
| 30 | Pluronic F68 | 70 | 95% Ethanol |
| 50 | Pluronic F68 | 50 | Acetone:ethanol: water (50:40:10) |
| **Polymethacrylates** | | | |
| 10 | Eudragit^{®}E-100 | 90 | 95% Ethanol |
| 30 | Eudragit^{®}E-100 | 70 | 95% Ethanol |
| 10 | Eudragit^{®}L-100-55 | 90 | 95% Ethanol |
| 30 | Eudragit^{®}L-100-55 | 70 | 95% Ethanol |
| **Compositions comprising polymers of each class and one surfactant** | | | |
| 10 | HPC / vitamin E TPGS | 48/2 | 95% Ethanol |
| 10 | HPC / Solutol HS 15 | 48/2 | 95% Ethanol |
| 10 | HPMC / vitamin E TPGS | 48/2 | 95% Ethanol |
| 10 | HPMC / Solutol HS 15 | 48/2 | 95% Ethanol |
| 10 | HPMC-AS / vitamin E TPGS | 48/2 | 95% Ethanol |
| 10 | HPMC-AS / Solutol HS 15 | 48/2 | 95% Ethanol |
| 10 | PVPK30 / vitamin E TPGS | 48/2 | 95% Ethanol |
| 10 | PVPK30 / Solutol HS 15 | 48/2 | 95% Ethanol |
| 10 | PEG6000 / vitamin E TPGS | 48 / 2 | 95% Ethanol |
| 10 | PEG8000 / vitamin E TPGS | 48 / 2 | 95% Ethanol |
| 10 | Pluronic F68 / vitamin E TPGS | 48 / 2 | 95% Ethanol |
| 10 | Pluronic F68 / Solutol HS 15 | 48 / 2 | 95% Ethanol |
| 10 | Eudragit^{®}E-100 / vitamin E TPGS | 48 / 2 | 95% Ethanol |
| 10 | Eudragit^{®}E-100 / Solutol HS 15 | 48 / 2 | 95% Ethanol |
| 10 | Eudragit^{®}L-100-55 / vitamin E TPGS | 48 / 2 | 95% Ethanol |
| 10 | Eudragit^{®}L-100-55 / Solutol HS 15 | 48 / 2 | 95% Ethanol |

### EXAMPLE 2

This Example lists representative compositions of cyclodextrin - Imatinib Mesylate complexes (Table 2) and illustrates making the composition according to the invention.
The cyclodextrin - Imatinib Mesylate complexes are prepared using HTS technology following the instructions described in Example 1 with minor modifications as follows. An amount of Imatinib Mesylate, of each cyclodextrin, and of each additional excipient is dissolved in ethanol 95% to produce individual stock solutions of each component. Then, an adequate volume of stock solution of Imatinib Mesylate is added to each well, followed by evaporation to dryness, to yield the desired amount of Imatinib Mesylate in each well. An adequate volume of cyclodextrin stock solution (β-cyclodextrin or hydroxypropyl-β-cyclodextrin) and additional excipient stock solution (if present in the formulation) is added to each well, followed by evaporation to dryness. The 96-well plate is scanned using XRPD to monitor the presence of crystalline Imatinib Mesylate both prior and after storage for 1 mo. at 40°C/75% RH.

**Table 2 - Representative compositions of cyclodextrin - Imatinib Mesylate complexes**

| Imatinib Mesylate (wt%) | β-cyclodextrin (wt%) | Hydroxypropyl-β- cyclodextrin (wt%) | Additional excipients | Additional excipient (wt%) |
|---|---|---|---|---|
| 10 | 90 | 0 | - | - |
| 30 | 70 | 0 | - | - |
| 10 | 45 | 0 | PVPK30 | 45 |
| 30 | 25 | 0 | PVPK30 | 25 |
| 10 | 45 | 0 | HPMC | 45 |
| 30 | 25 | 0 | HPMC | 25 |
| 10 | 0 | 90 | - | - |
| 30 | 0 | 70 | - | - |
| 10 | 0 | 45 | HPMC | 45 |
| 30 | 0 | 25 | HPMC | 25 |
| 10 | 0 | 80 | Vitamin E TPGS | 10 |
| 30 | 0 | 60 | Vitamin E TPGS | 10 |

### EXAMPLE 3

This Example lists representative dry co-milled Imatinib Mesylate - excipient compositions (Table 3) and illustrates making the composition according to the invention.
Imatinib Mesylate (amorphous form) is co-milled with excipients in the dry state as follows. A blend (2.5 g total amount) of Imatinib Mesylate and excipient is mixed with zirconia beads (5 g, 3 mm in diameter) for 10 min in a bench-top turbula prior starting the milling experiment. An adequate amount of the Imatinib Mesylate/excipient/zirconia beads blend (3.75 g) is then transferred to the milling vessel of a vibration mill, and milled for 2 hours at ambient temperature and 1000 rpm. The final powder is analyzed by XRPD both prior and after storage for 1 mo. at 40°C/75% RH.

**Table 3 - Representative dry co-milled Imatinib Mesylate - excipients compositions**

| Imatinib Mesylate (wt%) | Excipient type | Excipient (wt%) |
|---|---|---|
| 30 | HPMC | 70 |
| 30 | PVPK30 | 70 |
| 50 | PVPK30 | 50 |
| 30 | Precipitated calcium silicate (Zeopharm^{®}600) | 70 |

### EXAMPLE 4

This Example illustrates a wet co-milled Imatinib Mesylate - excipient composition and illustrates-making the composition according to the invention.
Imatinib Mesylate (amorphous form) is co-milled with excipients in fractionated coconut oil (Miglyoil 812^{®}) as follows. The following excipients are added to a glass vessel containing 80g of Miglyoil 812^{®}:
1.92g of Pluronic F-68
0.08g of sodium dodecyl sulfate (SDS)
The resultant mixture is stirred at ambient temperature with a conventional propeller mixer until an homogeneous suspension of the excipients is obtained. Subsequently, 2g of Imatinib Mesylate are added to the suspension, followed by stirring until an homogeneous dispersion is obtained. The resultant suspension is then transferred to the glass vessel of a DYNO-MILL and 170 g of glass beads (0.75 - 1 mm) are added. Milling is processed for 6 h under the following operating conditions: stirrer speed 3200 rpm, jacket cooling with water.
The final co-milled product is analyzed by XRPD both prior and after storage for 1 mo. at 40°C/75% RH.

## Claims

1. A stabilized amorphous form of Imatinib mesylate.

2. A pharmaceutical composition comprising a formulation principle that stabilizes the amorphous form of Imatinib mesylate and amorphous Imatinib mesylate, optionally together with at least one pharmaceutically acceptable carrier.

3. The pharmaceutical composition according to claim 2, wherein the formulation principle is selected from solid dispersions, cyclodextrin complexes, and co-milling with selected excipients.

4. A pharmaceutical composition according to claim 3, wherein the formulation principle is a solid dispersion.

5. A pharmaceutical composition according to claim 3, wherein the formulation principle is a cyclodextrin complex.

6. The pharmaceutical composition according to any one of claims 2 to 5 which is a capsule.

7. The capsule according to claim 6 which contains between an amount of stabilized amorphous Imatinib mesylate which corresponds to 50 mg and 200 mg of Imatinib mesylate.

8. The capsule according to claim 7, wherein the shell contains gelatine.

9. The capsule according to claim 7, wherein the shell contains titanium dioxide.

10. The capsule according to claim 7, wherein the shell contains red iron oxide.

11. The capsule according to claim 7, wherein the ratio of weight of capsule fill to capsule shell is between about 100:25 and 100:50.

12. The capsule according to claim 7, wherein the ratio of weight of capsule fill to capsule shell is between 100:30 and 100:40.

13. The pharmaceutical composition according to any one of claims 2 to 5, which is a tablet.

14. The tablet according to claim 13 comprising an amount of stabilized amorphous Imatinib mesylate which corresponds to 100 mg, 400 mg or 800 mg of Imatinib mesylate.

15. The use of a stabilized amorphous form of Imatinib mesylate according to claim 1 for the preparation of a medicament for the treatment of a disease selected from metastatic, inoperable GIST, advanced chronic myeloid leukemia, newly diagnosed chronic myeloid leukemia, pediatric Philadelphia chromosome-positive chronic myeloid leukemia, Philadelphia chromosome-positive acute lymphocytic leukemia (ALL), glioblastoma multiforme, dermatofibrosarcoma protuberans (DFSP), hypereosinophilic sindrome (HES), and chronic myelomonocytic leucemia (CMML).

16. Method of treating a disease selected from metastatic, inoperable GIST, advanced chronic myeloid leukemia, newly diagnosed chronic myeloid leukemia, pediatric Philadelphia chromosome-positive chronic myeloid leukemia, Philadelphia chromosome-positive acute lymphocytic leukemia (ALL), glioblastoma multiforme, dermatofibrosarcoma protuberans (DFSP), hypereosinophilic sindrome (HES), and chronic myelomonocytic leucemia (CMML in a warm-blooded animal in need thereof comprising administering to the animal a stabilized amorphous form of Imatinib mesylate according to claim 1 in a quantity which is therapeutically effective against the respective disease.

17. The use of a formulation principle which stabilizes the amorphous form of Imatinib mesylate as an intermediate for the preparation of a pharmaceutical composition comprising the amorphous form of Imatinib mesylate.
